# EUROPÄISCHE PATENTSCHRIFT

(11) **EP 2 654 740 B1**
(45) Veröffentlichungstag und Bekanntmachung des Hinweises auf die Patenterteilung: **11.02.2015**
(21) Anmeldenummer: 11805497.2
(22) Anmeldetag: 21.12.2011
(51) Int. Cl.: A61K 31/135, A61K 33/22

(54) **ANTIBAKTERIELL UND ANTIMYKOTISCH WIRKENDE BIPHENYLYL-VERBINDUNGEN**
BIPHENYLYL COMPOUNDS HAVING ANTIBACTERIAL AND ANTIMYCOTIC ACTIVITY
COMPOSÉS BIPHÉLIQUES À ACTIVITÉ ANTIBACTÉRIENNE ET ANTIMYCOTIQUE

(30) Priorität: 21.12.2010 DE 102010055322
(43) Veröffentlichungstag der Anmeldung: 30.10.2013
(73) Patentinhaber: Christian-Albrechts-Universität zu Kiel, 24118 Kiel (DE)
(72) Erfinder: CLEMENT, Bernd, 24106 Kiel (DE); FURKERT, Franz, 24211 Preetz (DE); GERIG, Britta, 81375 München (DE); HEBER, Dieter, 24113 Molfsee (DE)
(74) Vertreter: Steinecke, Peter
(86) Internationale Anmeldenummer: PCT/EP2011/073601
(87) Internationale Veröffentlichungsnummer: WO 2012/085092

(56) Entgegenhaltungen:
- WO-A1-2008/003299
- DE-A1- 2 551 591
- US-A- 4 055 664
- US-A- 5 177 067
- US-B2- 6 710 080

## Beschreibung

Die Erfindung betrifft antibakteriell und antimykotisch wirkende Substanzen und deren Verwendung zur Herstellung von Arzneimitteln.

Bakterielle Infektionen sind trotz aller wissenschaftlichen Fortschritte eine bleibende Bedrohung für die Menschheit.

Die heutige Bedrohung resultiert vorrangig aus der Zunahme von Resistenzen gegenüber handelsüblichen Antibiotika und der geringen Anzahl neuer Wirkstoffe [Gould, I. M., "Antibiotic resistance: the perfect storm" International Journal of Antimicrobial Agents 2009, 34 (3), 2-5].

WO 2008/003299 beschreibt basische Acetophenone als Hemmstoffe von NO-Synthasen und deren Verwendung zur Herstellung von Arzneimitteln.

Ein besonderes Problem stellen dabei die Methicillin resistenten Keime dar; ist ein Staphylococcus aureus gegen Methicillin resistent (sog. MRSA), so zeigen sich häufig auch Resistenzen gegen Antibiotika anderer Gruppen (Chinolone, Tetracycline, Aminoglykoside, Erythromycin, Sulfonamide).

Es ist daher Aufgabe der Erfindung, neue Verbindungen bereitzustellen, die gegen verschiedene Bakterien und Pilze, insbesondere gegen solche, die bereits Multiresistenz zeigen, wirksam sind und sich dabei als pharmazeutische Wirkstoffe eignen.

Es hat sich gezeigt, dass basische Biphenyle der allgemeinen Formel 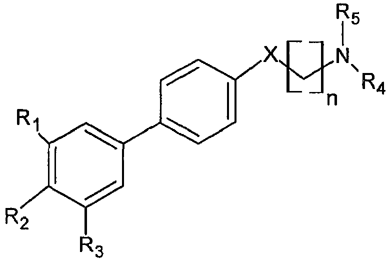 wobei X eine Methylengruppe (CH₂) oder eine Carbonylgruppe (C=O), R₁, R₂ und R₃ Wasserstoff, eine Alkylgruppe mit einer Kettenlänge von 1 - 4 Kohlenstoffatomen, (z. B. Methyl (CH₃)-, Ethyl (CH₂CH₃)-, Propyl (CH₂CH₂CH₃)-, Isopropyl (CHCH₃CH₃)-, Butyl (CH₂CH₂CH₂CH₃)-, Isobutyl (CHCH₃CH₂CH₃)- oder tert.Butyl (CCH₃CH₃CH₃)- Gruppe) eine Alkoxygruppe mit einer Kettenlänge von 1 - 3 Kohlenstoffatomen (z.B. eine Methoxy (OCH₃)-, Ethoxy (OCH₂CH₃)- Propoxy (OCH₂CH₂CH₃)- oder Isopropoxy (OCHCH₃CH₃)- Gruppe) oder ein Halogen (z.B. Fluor (F), Chlor (Cl), Brom (Br) oder Jod (J)),
R₄ und R₅ Wasserstoff oder eine Alkylgruppe mit einer Kettenlänge von 1 - 4 Kohlenstoffatomen (z. B. Methyl (CH₃)-, Ethyl (CH₂CH₃)-, Propyl (CH₂CH₂CH₃)-, Isopropyl (CHCH₃CH₃)-, Butyl (CH₂CH₂CH₂CH₃)-, Isobutyl (CHCH₃CH₂CH₃)- oder tert.Butyl (CCH₃CH₃CH₃)- Gruppe)
und
n = 3-6 sein kann, sowie die Salze, Hydrochloride und Tautomere dieser Verbindungen eine antimikrobielle und antimykotische Wirkung auch gegen Methicillin resistente Keime aufweisen. Daher eignen sich die vorstehend beschriebenen und in den nachfolgenden Ausführungsformen illustrierten Bisphenyle zur Hemmung des Wachstums von Bakterien und/oder Pilzen. Dementsprechend betrifft die vorliegende Erfindung die Verwendung der erfindungsgemäßen Stoffe als antibakterielles und/oder antimykotisches Arzneimittel.

Tabelle 1 zeigt die Werte der minimalen Hemmkonzentration (MHK) der erfindungsgemäßen Substanzen. Zum Vergleich wurde die Substanz GG20-3, die sich von den erfindungsgemäßen Substanzen durch eine kürzere Methylengruppenkette (n = 2) unterscheidet, sowie die bekannten Antibiotika Vancomycin und Tetracyclin ebenfalls vermessen.

### Ermittlung der minimalen Hemmkonzentration (MHK)

Die minimale Hemmkonzentration ist die niedrigste Konzentration der geprüften Substanz, die vollständig das Wachstum des jeweiligen Testorganismus hemmt, d.h. bei der nach der Inkubation keine Trübung messbar ist. Die jeweilige Bestimmung ist gültig, wenn in der Wachstumskontrolle (DMSO und Medium) deutliche Trübung festgestellt wird.

### Abkürzungen

Staphylococcus aureus S. a., Methicillin-resistenter Staphylococcus aureus MRSA, Escherichia coli E. c., Pseudomonas aeroginosa P. a., Staphylococcus epidermis S. e., Candida albicans C. a., Enterococcus hirae E. h., Aspergillus niger A. n., Aspergillus fumigatus A. f., n. b. nicht bestimmt.
Tabelle 1 (1 Teil: MHK-Werte (µg/ml)*

| Substanz | Code | S.a. | MRSA | E.c. | P.a. | S.e. |
|---|---|---|---|---|---|---|
| 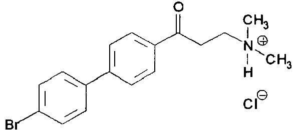 | GG20-3 | 32 | 32 | >64 | >64 | 16 |
| 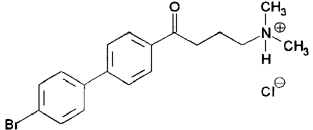 | GG20-4 | 4-8 | 8 | >64 | >64 | 4 |
| 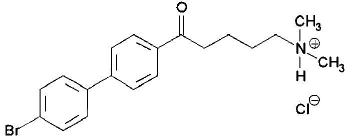 | GG20-5 | 16 | 16 | n. b. | n. b. | n. b. |
| 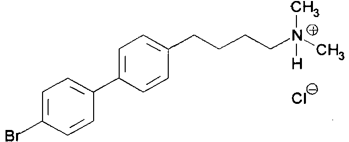 | GG28 | 13 (3) | 18(3) | n. b. | n. b. | n. b. |
| Vancomycin | | | <2 (2) | | | |
| Tetracyclin | | | 64 | | | |

| | | | | | | |
|---|---|---|---|---|---|---|
| *bei Ermittlung aus mehreren Testen: Angabe von gerundeten Mittelwerten der logarithmierten Endpunkte, Anzahl der Teste in Klammern. | | | | | | |

**Tabelle 1 (2 Teil): MHK-Werte (µg/ml)***

| Substanz | Code | C. a. (Hefe) | E.h | A. n. | A. f. |
|---|---|---|---|---|---|
| 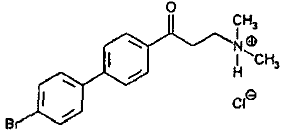 | GG20-3 | 64 10(3) | n. b. | n. b. | n. b. |
| 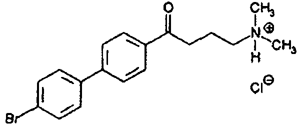 | GG20-4 | 4 11(5) | 16 | 64 | 8 |
| 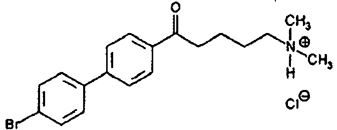 | GG20-5 | 8 | n. b. | n. b. | n.b. |
| 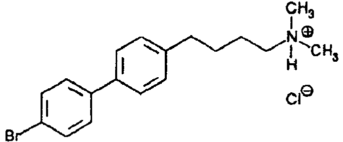 | GG 28 | 8(2) | n. b. | n. b. | n.b. |

| | | | | | |
|---|---|---|---|---|---|
| *bei Ermittlung aus mehreren Testen: Angabe von gerundeten Mittelwerten der logarithmierten Endpunkte, Anzahl der Teste in Klammern. | | | | | |

Es zeigt sich in allen Fällen ein unerwartet deutlicher Anstieg der Wirksamkeit beim Übergang der Verbindung GG20-3 mit n = 2 zu den Verbindungen, bei denen die Kette um eine oder mehrere Methylengruppen verlängert wurde (n≥3).

Des Weiteren ist die hohe Wirksamkeit der erfindungsgemäßen Verbindungen gegen verschiedene Keime, insbesondere auch MRSA zu erkennen.

Ein besonderer Vorteil der erfindungsgemäßen Substanzen ist ihre gute Wasserlöslichkeit, dieses stellt bei der Anwendung als Arzneimittel einen großen Vorteil dar, da zum Einen die orale Verfügbarkeit des Wirkstoffes erhöht ist zum Anderen eine Gabe des Arzneimittels durch Injektion wässriger Lösungen möglich ist. In einem Testversuch konnten 100 mg der Verbindung GG20-4 (siehe Tab. 1) in 10 ml destillierten Wasser gelöst werden, wobei der Fachmann weiß, dass Arzneimittel typischerweise in Kombination mit pharmazeutisch geeigneten Hilfsstoffen appliziert werden.

Als ein charakteristisches Beispiel für eine der erfindungsgemäßen Substanzen, die sowohl antibakteriell als auch antimykotisch wirkt, ist 4'-(4-Bromphenyl)-4-dimethylaminobutyro-phenon-Hydrochlorid 1 (GG20-4) mit n = 3 zu nennen:

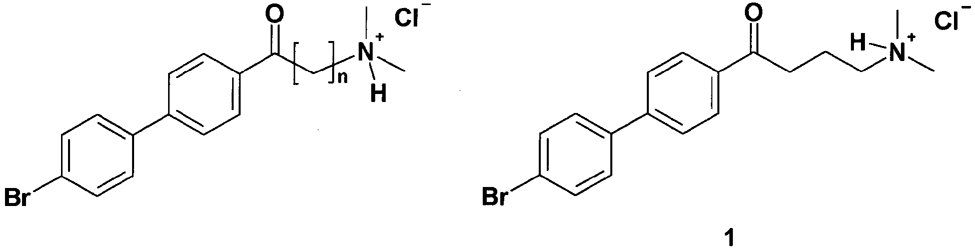

Als ein weiteres Beispiel für eine der erfindungsgemäßen Substanzen ist die Verbindung 4-Brom-4'-(4-dimethylaminobutyl)biphenylhydrochlorid 2 (GG28) zu nennen. Diese Substanz ist nicht literaturbekannt und wirkt ebenfalls sowohl antibakteriell als auch antimykotisch.

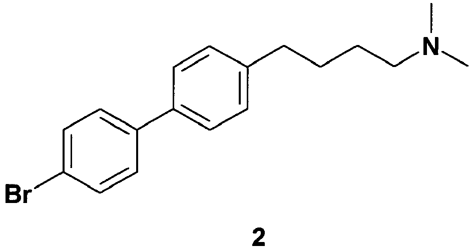

Die Synthesen der erfindungsgemäßen antimikrobiell und antimykotisch wirksamen Stoffe sind in Schema 1 und 2 dargestellt. Sie können nach gängigen Methoden hergestellt werden. Die Vergleichs-Verbindungen mit der Kettenlänge n = 2 können durch Umsetzung teils substituierter Acetophenone mit Dimethylammoniumchlorid und Paraformaldehyd im Sinne einer Mannich-Reaktion erhalten werden. Verbindungen mit der Kettenlänge n = 3 wurden durch Reaktion von substituierten 4-Chlor-butyrophenonderivaten mit Dimethylamin im Sinne einer nucleophilen Substitution erhalten. Diese Reaktion ist im Folgenden beispielhaft für die Verbindung 4'-(4-Bromphenyl)-4-dimethylaminobutyrophenonhydrochlorid 1 (GG20-4) angegeben:

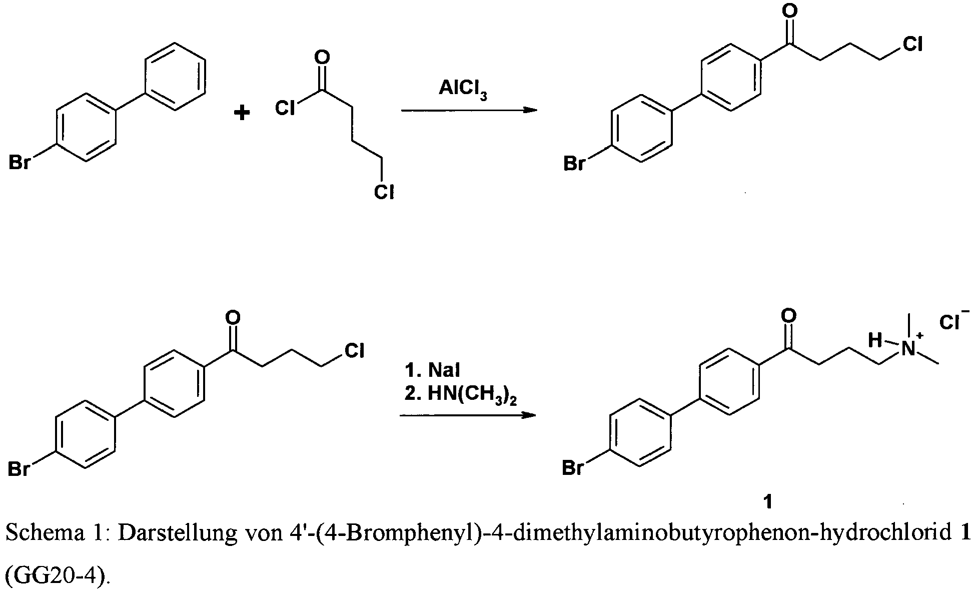

Die Verbindungen mit der Kettenlänge n = 4 wurden analog durch Reaktion von substituierten 5-Chlor-valerianophenonderivaten mit Dimethylamin im Sinne einer nucleophilen Substitution erhalten.

Die Verbindung 2 wurde ausgehend von Verbindung 1 im Sinne einer Wolff-Kishner-Reduktion nach der Huang-Minlon-Variante gewonnen:

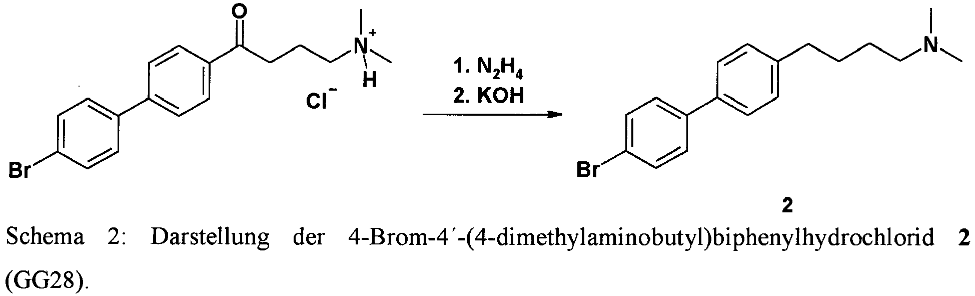

Die erfindungsgemäßen basischen Biphenyle stellen somit hochwirksame Hemmstoffe des Wachstums von Bakterien und Pilzen dar. Sie sind leicht und kostengünstig über Standardmethoden und in hoher Reinheit zu synthetisieren. Dementsprechend stellt die vorliegende Erfindung Verbindungen zur Hemmung des Wachstums von Bakterien und/oder Pilzen und dementsprechend Arzneimittel zur Behandlung von Infektionen von Bakterien und/oder Pilzen bereit.

Die Untersuchungen von analogen Verbindungen mit einem Linker aus drei, vier und fünf Kohlenstoffatomen auch ohne Ketofunktion ergeben, dass deren antibakterielle Wirkung von der Länge der Seitenkette abhängig ist. Andererseits führt der Ersatz der Dimethylaminodurch eine *N*-Phenylpiperazinogruppe zur Wirkungslosigkeit. In einem Testversuch konnte ein MHK-Wert für 4'-(4-Bromphenyl)-4-(*N'*-phenylpiperazino)butyrophenon-hydrochlorid nicht ermittelt werden, da diese Substanz das Wachstum von Staphylococcus aureus auch nach Zugabe höherer Konzentrationen nicht hemmte.

Erreger in der Wachstumsphase werden mit niedrigeren Konzentrationen getötet als solche in der Ruhephase. In einem Testversuch wurden die Testkeime einmal in der Exponentialphase ihres Wachstums (18 h vor Inkubation) eingesetzt im Vergleich zu einem Inokulum, das erst nach 48 h (Ruhephase) abgeerntet und eingesetzt wurde. Nicht mehr so teilungsfähige Zellen zeigten eine 2 - 3 fach höhere MHK als die aktiven Zellen. Damit ergaben sich Anzeichen, dass Ruhephasezellen weniger empfindlich sind.

Wie aus den vorstehenden Erläuterungen und den Ausführungsbeispielen ersichtlich, eignen sich die erfindungsgemäßen Stoffe auch als Laborreagenzien um das Wachstum von Bakterien und/oder Pilzen in Zellkultur zu hemmen, ähnlich wie dies für das Selektionsmarkersystem Neomycin und Neomycin-Phosphotransferase System beschrieben ist.

Die erfindungsgemäße Zubereitung wird anhand von Ausführungsbeispielen näher erläutert.

### Ausführungsbeispiel 1

### 4'-(4-Bromphenyl)-4-dimethylaminobutyrophenon-hydrochlorid 1

25,7 mmol (6 g) 4-Brombiphenyl, 25,7 mmol (3,6 g) 4-Chlorbuttersäurechlorid und 32,2 mmol (4,2 g) Aluminiumtrichlorid werden in einen 250 mL Kolben eingewogen, bevor man 50 mL trockenes Dichlormethan ergänzt. Nach Rühren über Nacht bei Raumtemperatur wird der Ansatz auf Eis gegeben und ausgefallenes Aluminiumhydroxid mittels konzentrierter Salzsäure in Lösung gebracht. Anschließend wird die organische Phase abgetrennt und so oft mit Wasser extrahiert, bis die Wasserphase einen neutralen pH-Wert aufweist. Man trocknet nun die organische Phase über Na₂SO₄, filtriert und entfernt das Lösungsmittel im Vakuum. Der Rückstand wird aus einem Gemisch von Cyclohexan / Ethylacetat umkristallisiert.

1,2 mmol (500 mg) des erhaltenen reinen Produktes werden mit 1,2 mmol (184 mg) NaI in 5 mL Ethylmethylketon versetzt und 1 ½ Stunden bei 90 °C auf dem Ölbad erhitzt. Anschließend entfernt man das Lösungsmittel im Vakuum, ergänzt 1 mL Dimethylamin sowie 30 mL absoluten Ethanol und erhitzt für weitere 8 Stunden am Rückfluss. Nach Entfernen des Lösungsmittels im Vakuum wird zu dem Rückstand Eis gegeben und das ausgefallene Rohprodukt abgesaugt. Umkristallisation aus Cyclohexan / Ethylacetat ergibt 1 in einer Ausbeute von 82 % (377 mg); Schmp. = 224 °C.

¹H-NMR-Daten (300 MHz, DMSO-*d*₆): 1,94 (m._{;} 2H, -CH₂-), 2,8 (s, 6H, N(CH₃)₂), 3,15 (m, 4H, COCH₂-; -CH₂NH(CH₃)₂), 7,70 (m, 4H, Ar-H); 7,85 (d, ³*J* = 8,7 Hz; 2H, H-3', H-5'). 8,04 (d, *³J*= 8,7 Hz; 2H, H-2', H-6'), 9.21 (bs, 1 H, NH). ¹³C-NMR-Daten (75 MHz, DMSO-*d₆):* 18,4 (C-3), 35,0 (C-2), 42,0 (2 x CH₃), 56,1 (C-4), 122,0 (C-4"), 126,8 (2C, C-3', C-5'), 128,6 (2C, C-2', C-6'), 129,0 (2C, C-2"; C-6"), 131,9 (2C, C-3", C-5"), 135,5 (C-1"), 138,0 (C-1'), 143,2 (C-4'), 198,2 (CO).

### Ausführungsbeispiel 2

### 4-Brom-4'-(4-dimethylaminobutyl)biphenylhydrochlorid 2

In einen 100 mL Kolben werden 0,02 mol (6,96 g) 1, 5 mL Hydrazin-Monohydrat und 60 mL absoluter Ethanol gegeben. Anschließend wird am Rückfluss erhitzt, bis eine vollständige Lösung eintritt (12 h). Dann werden im Vakuum 40 mL Ethanol entfernt und 40 mL Triglykol sowie 10 g Kaliumhydroxid und 3 mL Hydrazin Monohydrat ergänzt. Man erhitzt eine weitere Stunde auf 80 °C (Gasentwicklung!), bevor die Temperatur hochreguliert wird, bis das Thermometer 200 bis 220 °C anzeigt. Dann wird abgekühlt, der resultierende Niederschlag abgesaugt und aus Ethanol umkristallisiert. Ausbeute 85 % (5,6 g); Schmp. = 245 °C.

¹H-NMR-Daten (300 MHz, CDCl₃): 1,39 (m, 2H, -CH₂-), 1,62 (m, 2H, -CH₂-), 2,27 (s, 6H, N(CH₃)₂), 2,36 (t, *³J* = 8,4 Hz, 2H, -CH₂NH(CH₃)₂), 2,55 (t, *³J* = 8,4 Hz, 2H, Ar-CH₂-), 7,18 (d, *³J* = 8,7 Hz; 2H, H-2', H-6'); 7,42 (m, 4H, H-3', H-5', H-2", H-6"), 7,48 (d, *³J* = 8,7 Hz; 2H, H-3", H-5"). ¹³C-NMR-Daten (75 MHz, CDCl₃): 27,4 (C-3), 29,0 (C-2), 35,7 (C-1), 45,9 (2 x CH₃), 59,1 (C-4), 122,0 (C-4"), 127,7 (2C, C-3', C-5'), 128,7 (2C, C-2', C-6'), 130,1 (2C, C-2"; C-6"), 132,2 (2C, C-3", C-5"), 133,7 (C-4'), 135,5 (C-1"), 137,6 (C-1').

Die Schmelzpunkte der synthetisierten Substanzen wurden mit der Schmelzpunktapparatur Büchi 510 Gerät und Mikroheiztisch Thermovar (Firma Reichert) aufgenommen. Die Aufnahme von NMR-Spektren erfolgte mit dem Kernresonanzspektrometer Bruker ARX 300 und von IR-Spektren (als KBr-Presslinge) mit einem Perkin-Elmer FT-IR 16 PC Spectrometer. Die Massenspektren wurden mit einem Gerät vom Typ Hewlett-Packard 5989 aufgenommen. Elementaranalysen wurden im Institut für Anorganische Chemie der CAU zu Kiel mittels eines CHNS-Analysators der Firma Hekatech GmbH bestimmt. Soweit nicht anders angegeben, wurden die Chemikalien einschließlich Vancomycin-HCl und Tetracyclin-HCl in höchster Reinheit bei der Firma Sigma-Aldrich GmbH bezogen.

Die minimalen Hemmkonzentrationen der erfindungsgemäßen Substanzen gegen verschiedene Infektionskeime, u. a. gegen den Methicillin-resistenten Staphylococcus aureus, wurden mit der Bouillon-Mikrodilutionsmethode in Übereinstimmung mit der Vorschrift M07-A8 des Clinical and Laboratory Standards Institute (Pennsylvania, USA) bestimmt.

### Testorganismen

### (Bezugsquelle: Deutsche Sammlung für Mikroorganismen und Zellkulturen, Braunschweig)

Enterococcus hirae ATCC 10541, Staphylococcus epidermidis ATCC 12228, Staphylococcus aureus ATCC 6538, Methicillin-resistenter Staphylococcus aureus ATCC 33592, Bacillus cereus ATCC 11778, Candida albicans ATCC 10231, Aspergillus niger ATCC 16404, Aspergillus fumigatus ATCC 9197.

### Testgefäße

Sterile 96-Well-Plastikmikrotiterplatten mit abgerundeten Böden der Testvertiefungen (Wells; BRANDplates^{™}, Ref. 781960).

### Anzucht der Testorganismen

In sterilen Schrägagarröhrchen, Bakterien mit Caseinpepton-Sojamehlpepton-Agar (Merck, Art. Nr. 1.05458.05), Inkubation 18-24 h bei 34°C, Pilze mit Sabouraud 4 % Glucose Agar (Merck 1.05438.05), Inkubation Candida albicans 18-24 hrs bei 34°C, Schimmelpilze bei 22°C 7 Tage.

Die Langzeitkulturen der Testorganismen werden auf Schrägagar bei 22°C inkubiert und nach 4 Wochen jeweils auf frisches Medium überimpft. Nach jeder fünften Kulturpassage bzw. bei Feststellung von Unreinheiten wird die entsprechende Kultur verworfen und aus Lyophilisat neu angezüchtet.

### Herstellung der Inokula

Von den Schrägagarkulturen werden durch Abschwemmen mit 0,9 %iger steriler NaCl-Lösung Suspensionen hergestellt. Deren Trübung wird durch Verdünnung photometrisch auf die Trübung entsprechend des Mc Farland-Standards 0,5 eingestellt.

Die Bakteriensuspensionen werden danach noch einmal im Verhältnis 1:10 verdünnt, die Pi-Suspensionen der Pilze bleiben unverdünnt.

Die Keimzahl in den Inokula ist so eingestellt, dass sich nach Beimpfung der Testwells darin jeweils ca. 5 x 10⁵ koloniebildende Einheiten je Milliliter befinden.

### Herstellung der Antibiotikastammlösungen

Die für einen Test benötigte Menge einer zu prüfenden Substanz wird auf einer Mikrowaage in ein Eppendorf-Reaktionsgefäß eingewogen und in einem bestimmten Volumen Dimethylsulfoxid gelöst, so dass die Konzentration der resultierenden Lösung dem 21-fachen der höchsten endgültigen Testkonzentration entspricht. Diese Stammlösung wird anschließend in Serie im Verhältnis jeweils 1 ad 2 mit DMSO verdünnt, um insgesamt sieben verschiedene Konzentrationen in den Wells einer Vorverdünnungsplatte zu erhalten. In die achte Vertiefung wird nur DMSO pipettiert.

### Beschickung der Platten

In jedes der 96 Wells einer Testplatte werden mit einer Achtkanalpipette (Socorex 50-200µl) für Bakterien 95 µl steriles Mueller Hinton II Medium (Cation adjusted, BBL^{™} Ref 212322), für Pilze steriles Sabouraud 2 % Glucose Medium (Difco^{™} Ref 238230) gegeben. Die Testvertiefungen der Platten werden mit je 5 µl der zu prüfenden Lösungen versehen (Achtkanalpipette, Eppendorf-Research, 5-10µl), in dem diese aus den Vorverdünnungen so überführt werden, dass in Horizontalreihe A die höchste Konzentration und bis Reihe G die jeweils um die Hälfte verminderte Konzentration der Prüfsubstanz und in Reihe H nur DMSO pipettiert wird. Anschließend werden die Inokula mit Hilfe der Achtkanalpipette hinzugegeben, je Testvertiefung ebenfalls 5 µl. In einem Test werden mindestens zwei maximal vier Reihen mit der gleichen Substanz und der gleichen Keimart beimpft. Die Konzentrationen der Prüfsubstanz betragen jeweils 128 µg/ml bis 2 µg/ml.

### Auswertung und Inkubation der Mikrotiterplatten

Die präparierten Platten werden in ein Mikrotiterplattenlesegerät (Anthos htIII), das mit einem Drucker verbunden ist, überführt. In diesem Gerät werden die Platten vor jeder Messung 60 Sekunden mit hoher Frequenz geschüttelt und dann bei Licht der Wellenlänge 590 nm die Absorptionen der Proben gemessen.

Nach Ermittlung der Anfangswerte Inkubation der Platten bei 34 °C für 16 bis 20 hrs im Falle der Bakterien und von Candida albicans, 68 bis 72 hrs. bei 34 °C im Falle der Schimmelpilze.

Anschließend werden die Mikrotiterplatten erneut wie oben beschrieben vermessen.

### Ermittlung der minimalen Hemmkonzentration (MHK)

Die minimale Hemmkonzentration ist die niedrigste Konzentration der geprüften Substanz, die vollständig das Wachstum des jeweiligen Testorganismus hemmt, d.h. bei der nach der Inkubation keine Trübung messbar ist. Die jeweilige Bestimmung ist gültig, wenn in der Wachstumskontrolle (DMSO und Medium) deutliche Trübung festgestellt wird.

## Patentansprüche

1. Stoff der allgemeinen Formel 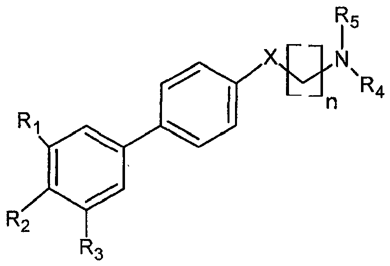 zur Verwendung als antibakterielles und/oder antimykotisches Arzneimittel, wobei
- X eine Methylen- oder eine Carbonylgruppe ist;
- R₁, R₂ und R₃ jeweils ausgewählt sind aus der Gruppe bestehend aus Wasserstoff, einer Alkylgruppe mit einer Kettenlänge von 1 - 4 Kohlenstoffatomen, einer Alkoxygruppe mit einer Kettenlänge von 1 - 3 Kohlenstoffatomen und einem Halogen;
- R₄ und R₅ jeweils ausgewählt sind aus der Gruppe bestehend aus Wasserstoff und einer Alkylgruppe mit einer Kettenlänge von 1 - 4 Kohlenstoffatomen; und
- n = 3 bis 6 ist.

2. Stoff zur Verwendung nach Anspruch 1, **dadurch gekennzeichnet, dass** der Stoff als Hydrochlorid, als Salz oder als Tautomer vorliegt.

3. Stoff zur Verwendung nach einem der vorhergehenden Ansprüche, **dadurch gekennzeichnet, dass** die Alkylgruppe eine Methyl (CH₃)-, eine Ethyl (CH₂CH₃)-, eine Propyl (CH₂CH₂CH₃)-, eine Isopropyl (CHCH₃CH₃)-, eine Butyl (CH₂CH₂CH₂CH₃)-, eine Isobutyl (CHCH₃CH₂CH₃)- oder eine tertiäre Butyl (CCH₃CH₃CH₃)- Gruppe ist.

4. Stoff zur Verwendung nach einem der vorhergehenden Ansprüche, **dadurch gekennzeichnet, dass** die Alkoxygruppe eine Methoxy (OCH₃)-, eine Ethoxy (OCH₂CH₃)-, eine Propoxy (OCH₂CH₂CH₃)- oder eine Isopropoxy (OCHCH₃CH₃)- Gruppe ist.

5. Stoff zur Verwendung nach einem der vorhergehenden Ansprüche, **dadurch gekennzeichnet, dass** das Halogen Fluor (F), Chlor (Cl), Brom (Br) oder Jod (J) ist.

6. Stoff zur Verwendung nach einem der vorhergehenden Ansprüche, **dadurch gekennzeichnet, dass** die Wasserlöslichkeit des Stoffs gleich oder größer als 1.000 mg/l ist.

7. Stoff zur Verwendung nach einem der vorhergehenden Ansprüche, **dadurch gekennzeichnet, dass** die Wasserlöslichkeit des Stoffs gleich oder größer als 5.000 mg/l ist.

8. Stoff zur Verwendung nach einem der vorhergehenden Ansprüche, **dadurch gekennzeichnet, dass** die Wasserlöslichkeit des Stoffs gleich oder größer als 10.000 mg/l ist.

9. Stoff zur Verwendung nach einem der vorgehenden Ansprüche, **dadurch gekennzeichnet, dass** der Stoff 4'-(4-Bromphenyl)-4-dimethylaminobutyrophenonhydrochlorid mit der Strukturformel 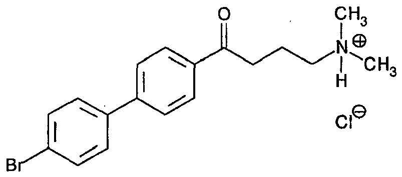 oder 4-Brom-4'-(4-dimethylaminobutyl)biphenylhydrochlorid mit der Strukturformel 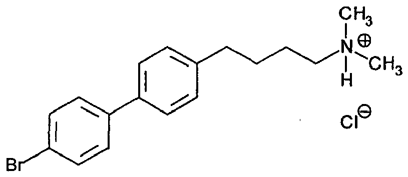 ist.

## Claims

1. A substance of the general formula 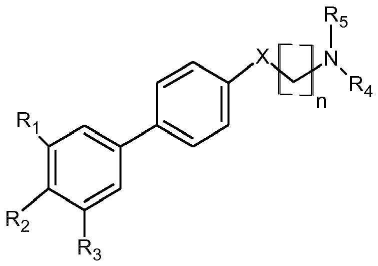 for use as antibacterial and/or antifungal drug, wherein
- X is a methylene or a carbonyl group;
- R₁, R₂ and R₃ are each selected from the group consisting of hydrogen, an alkyl group having a chain length of 1 - 4 carbon atoms, an alkoxy group with a chain length of 1 - 3 carbon atoms, and a halogen;
- R₄ and R₅ are each selected from the group consisting of hydrogen and an alkyl group with a chain length of 1 - 4 carbon atoms; and
- n = 3 to 6.

2. The substance for use of claim 1, wherein the substance is available as a hydrochloride, a salt, or as a tautomer.

3. The substance for use of any one of the preceding claims, wherein the alkyl group is a methyl (CH₃)-, an ethyl (CH₂CH₃)-, a propyl (CH₂CH₂CH₃)-, an isopropyl (CHCH₃CH₃)-, a butyl (CH₂CH₂CH₂CH₃)-, an isobutyl (CHCH₃CH₂CH₃)- or a tertiary butyl (CCH₃CH₃CH₃)- group.

4. The substance for use of any one of the preceding claims, wherein the alkoxy group is a methoxy (OCH₃)-, an ethoxy (OCH₂CH₃)-, a propoxy (OCH₂CH₂CH₃)- or an isopropoxy (OCHCH₃CH₃)- group.

5. The substance for use of any one of the preceding claims, wherein the halogen is fluorine (F), chlorine (Cl), bromine (Br), or iodine (I).

6. The substance for use of any one of the preceding claims, wherein the water-solubility of the substance is equal to or greater than 1,000 mg/l.

7. The substance for use of any one of the preceding claims, wherein the water-solubility of the substance is equal to or greater than 5,000 mg/l.

8. The substance for use of any one of the preceding claims, wherein the water-solubility of the substance is equal to or greater than 10,000 mg/l.

9. The substance for use of any one of the preceding claims, wherein the substance is 4'-(4-bromphenyl)-4-dimethylaminobutyrophenonehydrochloride with the structural formula 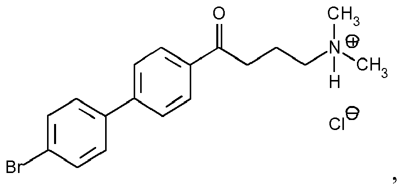 or is 4-brom-4'-(4-dimethylaminobutyl)biphenylhydrochloride with the structural formula

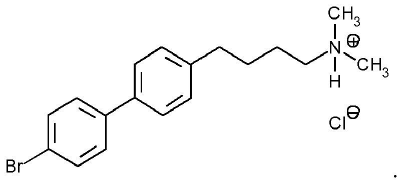

## Revendications

1. Substance de formule générale 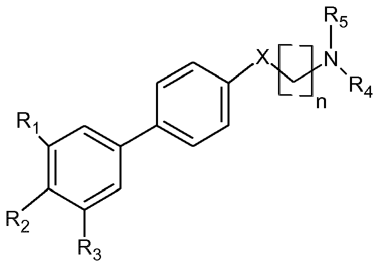 pour son utilisation comme médicament antibactérien et/ou antimycotique, dans laquelle
- X est un groupe méthylène ou carbonyle;
- R₁, R₂ et R₃ sont choisis respectivement dans le groupe comprenant un atome d'hydrogène, un groupe alkyle présentant une longueur de chaîne de 1 à 4 atomes de carbone, un groupe alcoxy présentant une longueur de chaîne de 1 à 3 atomes de carbone et un halogène;
- R₄ et R₅ sont choisis respectivement dans le groupe comprenant un atome d'hydrogène et un groupe alkyle présentant une longueur de chaîne de 1 à 4 atomes de carbone; et
- n = 3 à 6.

2. Substance pour son utilisation selon la revendication 1, **caractérisée en ce que** la substance se présente sous forme de chlorhydrate, de sel ou de tautomère.

3. Substance pour son utilisation selon l'une des revendications précédentes, **caractérisée en ce que** le groupe alkyle est un groupe méthyle (CH₃), un groupe éthyle (CH₂CH₃), un groupe propyle (CH₂CH₂CH₃), un groupe isopropyle (CHCH₃CH₃), un groupe butyle (CH₂CH₂CH₂CH₃), un groupe isobutyle (CHCH₃CH₂CH₃) ou un groupe butyle tertiaire (CCH₃CH₃CH₃).

4. Substance pour son utilisation selon l'une des revendications précédentes, **caractérisée en ce que** le groupe alcoxy est un groupe méthoxy (OCH₃), un groupe éthoxy (OCH₂CH₃), un groupe propoxy (OCH₂CH₂CH₃) ou un groupe isopropoxy (OCHCH₃CH₃).

5. Substance pour son utilisation selon l'une des revendications précédentes, **caractérisée en ce que** l'atome d'halogène est un atome de fluor (F), de chlore (Cl), de brome (Br) ou d'iode (I).

6. Substance pour son utilisation selon l'une des revendications précédentes, **caractérisée en ce que** la solubilité dans l'eau de la substance est supérieure ou égale à 1.000 mg/l.

7. Substance pour son utilisation selon l'une des revendications précédentes, **caractérisée en ce que** la solubilité dans l'eau de la substance est supérieure ou égale à 5.000 mg/l.

8. Substance pour son utilisation selon l'une des revendications précédentes, **caractérisée en ce que** la solubilité dans l'eau de la substance est supérieure ou égale à 10.000 mg/l.

9. Substance pour son utilisation selon l'une des revendications précédentes, **caractérisée en ce que** la substance est le chlorhydrate de 4'-(4-bromophényl)-4-diméthylaminobutyrophénone de formule développée 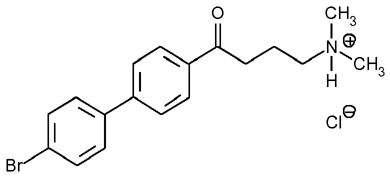 ou le chlohydrate de 4-bromo-4'-(4-diméthylaminobutyl)biphényle de formule développée
